# EUROPEAN PATENT SPECIFICATION

(11) **EP 1 616 950 B1**
(45) Date of publication and mention of the grant of the patent: **25.06.2008**
(21) Application number: 04727742.1
(22) Date of filing: 15.04.2004
(51) Int. Cl.: C12N 15/09, C07K 14/15, C07K 16/10, C07K 19/00, C12N 1/15, C12N 1/19, C12N 1/21, C12N 5/00, A61K 39/21, A61P 31/14, A61P 35/04

(54) **PEPTIDE HAVING HTLV-1-SPECIFIC CTL-INDUCING ACTIVITY**
PEPTID MIT HTLV-1-SPEZIFISCHER CTL-INDUZIERENDER AKTIVITÄT
PEPTIDE PRESENTANT UNE ACTIVITE INDUCTRICE DE CTL SPECIFIQUES DU HTLV-I

(30) Priority: 16.04.2003 JP 2003112138
(43) Date of publication of application: 18.01.2006
(73) Proprietor: Japan Science and Technology Agency, Kawaguchi-shi, Saitama 332-0012 (JP)
(72) Inventor: HARASHIMA, Nanae, c/o Hematology Br., NHLBI, NIH, MSC-1202 Bethesda, MD 20892-1202 (US); KANNAGI, Mari, Tokyo 1120002 (JP)
(74) Representative: Harding, Charles Thomas
(86) International application number: PCT/JP2004/005414
(87) International publication number: WO 2004/092373

(56) References cited:
- EP-A- 1 391 729
- WO-A-94/19473
- KOENIG SCOTT ET AL: "Characterization of MHC class I restricted cytotoxic T cell responses to tax in HTLV-1 infected patients with neurologic disease" JOURNAL OF IMMUNOLOGY, vol. 151, no. 7, 1993, pages 3874-3883, XP002410424 ISSN: 0022-1767
- KANNAGI M. ET AL.: 'Target epitope in the Tax protein of human T-cell leukemia virus type I recognized by class I major histocompatibility complex-restricted cytotoxic T cells' J. VIROL. vol. 66, no. 5, 1992, pages 2928 - 2933, XP000857308
- ARAI J. ET AL.: 'Identification of human telomerase reverse transcriptase-derived peptides that induce HLA-A24-restricted antileukemia cytotoxic T lymphocytes' BLOOD vol. 97, 01 May 2001, pages 2903 - 2907, XP002980964
- TAKENOYAMA M. ET AL.: 'Autologous tumor-specific cytotoxic T lymphocytes in a patient with lung adenocarcinoma: implications of the shared antigens expressed in HLA-A24 lung cancer cells' JPN. J. CANCER RES. vol. 89, 1998, pages 60 - 66, XP002980965
- GARBOCZI D.N. ET AL.: 'HLA-A2-peptide complexes: Refolding and crystallization of molecules expressed in Escherichia coli and complexed with single antigenic peptides' PROC. NATL. ACAD. SCI. USA vol. 89, 1992, pages 3429 - 3433, XP002131059
- HARASHIMA N. ET AL.: 'Graft-versus-Tax response in adult T-cell leukemia patients after hematopoietic stem cell transplantation' CANCER RESEARCH vol. 64, 01 January 2004, pages 391 - 399, XP002980966

## Description

The present invention relates to a peptide having HTLV-I-specific CTL-inducing activity that can induce cytotoxic T cells (CTL) having anti-tumor effect to human T-cell leukemia virus type I (HTLV-I) tumor, such as adult T-cell leukemia (ATL); a DNA encoding the peptide, or to a vaccine to induce immune response by using these and a diagnostic agent to examine immune function.

Adult T cell leukemia (ATL) is a T cell malignancy that develops in about 5% of human T-cell leukemia virus type-I (HTLV-I)-infected individuals, and is characterized by mostly CD4⁺ and CD25⁺ mature T lymphocyte phenotype, onset at middle age or later, immune suppression, and poor prognosis (see for example, Int. J. Cancer 45, 237-243, 1990; Blood 50, 481-492, 1977; Proc. Natl. Acad. Sci. USA 78, 6476-6480, 1981) . Clinical use of combination chemotherapy for ATL brought the four-year-overall-survival rate up to 8-12%, which is still lower than those of other types of leukemia (see for example, J. Clin. Oncol 6, 128-141, 1988; J. Clin. Onco 6, 1088-1097, 1988). Recently, hematopoietic stem cell transplantation (HSCT) has been applied to a limited number of ATL patients. Initial studies of autologous HSCT revealed frequent recurrence of ATL (see for example, Bone Marrow Transplant 23, 87-89, 1999). However, more recent reports revealed that allogeneic HSCT could produce better results, although there was also a risk of graft-versus-host diseases (GVHD) (see for example, Bone Marrow Transplant 27, 15-20, 2001). These reports strongly suggest that cellular immune reaction of donor against recipient, i.e. graft-versus-leukemia (GVL) effect, contributes to eradicating ATL cells, as observed in other types of leukemia.

It has been shown that allogeneic HSCT from human leukocyte antigen (HLA)-identical siblings can cause GVHD to some degree, and the minor histocompatibility antigen (mHA) in the recipient has been referred to as the target antigen of GVDH (see for example, N. Engl. J. Med. 334, 281-285, 1996) . Several mHA including the male-specific H-Y transplantation antigen (see for example, Science 269, 1588-1590, 1995), HA-1 antigen (see for example, Science 279, 1054-1057, 1998), CD31 molecule (see for example, N. Engl. J. Med. 334, 286-291, 1996; Br. J. Haematol 106, 723-729, 1999), and human platelet antigens (HPA) (see for example, Br. J. Haematol 106, 723-729, 1999; Blood 92, 2169-2176, 1998) have been suggested to be involved in GVHD. It is known that the probability of recurrence of leukemia after allogeneic HSCT increases when the graft has been depleted of T cells or the donor is a genetically identical twin, indicating that GVL effects are important in preventing the recurrence of leukemia (see for example, Blood 75, 552-562, 1990). Therefore, an augmentation of donor T cell response specific for mHA expressed in the recipient's hematopoietic cells but not in non-hematopoietic cells has been proposed as one strategy for inducing GVL effects without causing GVHD (see for example, Blood 93, 2336-2341, 1999). Tumor antigens, such as bcr/abl fusion protein and WT-1 which are specific for or over-expressed in tumor cells, are also candidates for the target antigens of GVL effects (see for example, Blood 95, 1781-1787, 2000; Blood 96, 1480-1489, 2000).

Host cellular immune responses against HTLV-I, especially outgrowth of cytotoxic T cells, can frequently be found in PBMC culture of asymptomatic HTLV-I-carriers and HTLV-I associated myelopathy/tropical spastic palsy (HAM/TSP) patients but infrequently in ATL patients (see for example, Leukemia 8 Suppll, S54-59, 1994; J. Immunol. 133, 1037-1041, 1984). Among HTLV-I antigens such as env, gag, pol, and pX gene products, it is known that Tax, being a pX gene product, is a dominant target antigen of HTLV-I-specific cytotoxic T lymphocytes (CTL) (see for example, Nature 348, 245-248, 1990; Int. Immunol. 3, 761-767, 1991). Tax is also known to play a critical role in HTLV-I leukemogenesis by accelerating cell growth and inhibiting apoptosis (see for example, Lancet 1, 1085-1086, 1987; J. Virol. 73, 7981-7987, 1999). These findings suggest that Tax-specific CTL may play a role in immune surveillance for leukemogenesis of HTLV-I-infected cells.

The present inventors have previously found major epitopes of CTL restricted by human HLA-A2 (see for example, J. Virol. 66, 2928-2933, 1992). However, HLA-A2 is positive to only 30-40% of the Japanese. Moreover, in a recently established animal model for HTLV-I infected T cell lymphoma, the present inventors demonstrated anti-tumor effect of Tax-specific CTL in vivo (see for example, Japanese Laid-Open Patent Application No. 2002-372532, J. Virol. 74, 428-435, 2000; J. Virol. 73, 6031-6040, 1999). In this model, T cell lymphomas, that can be fatal in nude rats if untreated, inoculated with syngeneic HTLV-I-infected cells could be eradicated by transferring fresh T cells from syngeneic immunocompetent rats vaccinated with either Tax-encoding DNA or peptides corresponding to a CTL epitope (see for example, J. Virol. 74, 9610-9616, 2000; J. Natl. Cancer Inst. 93, 1775-1783, 2001). However, it is unclear whether such observations in experimental models can be applied to humans, since HTLV-I-expression is extremely low in human ATL cells in the peripheral blood (see for example, non-patent document, Gann 73, 341-344, 1982; Int. J. Cancer 54, 582-588, 1993; Proc. Natl. Acad. Sci. USA 86, 5620-5624, 1989).

ATL is a neoplastic disease caused by HTLV-I infection which is highly carried in Japan, and as it is resistant to chemotherapeutic agent, it was deemed to be a malignant tumor having very poor prognosis. From various clinical observations and results of animal experiments, host cellular immunity, particularly anti-tumor effect of CTL has been suggested, it is revealed that HTLV-I Tax, which is a dominant target antigen of CTL has a function to promote tumorigenesis. Therefore, it is necessary to identify an epitope that recognizes CTL to develop a more specific and safe vaccine. However, CTL epitope having anti-tumor effect in human ATL-patient had not been identified. The object of the present invention is to provide a peptide having HTLV-I-specific CTL inducing activity that can induce cytotoxic T cells (CTL) having anti-tumor effect to human T-cell leukemia virus type I (HTLV-I) tumor such as adult T-cell leukemia (ATL), a DNA encoding the peptide, a vaccine to induce immune response by using these, and a diagnostic agent to examine the anti-tumor immune function.

The present inventors have investigated cellular immune responses of post-HSCT ATI. patients to HTLV-I infected T cells originating from the same pre-HSCT ATL patients. These HTLV-I infected cells were thought to have antigen originating from recipient, including target of GVL effect. The present inventors have found that post-HSCT PBMC respond actually to recipient-derived cells. However, most of response cells exhibited a strong reaction to HTLV-antigen, particularly to a limited number of Tax-epitope. Similar results were obtained from 2 post-HSCT ATL patients, and the donor of one patient was HTLV-I negative. From these observations, it has been revealed that graft-versus HTLV-I response was exhibited in post-HSCT ATL patients. During the process of these studies, the present inventors found the dominant epitope of HLA-A24-restricted CTL, and thus completed the present invention.

In one aspect, the present invention provides a peptide having HTLV-I-specific CTL-inducing activity consisting of the amino acid sequence shown in SEQ ID NO: 4.

In another aspect, the present invention provides a peptide having HTLV-I-specific CTL-inducing activity consisting of an amino acid sequence wherein one amino acid is deleted, substituted or added in the amino acid sequence shown in SEQ ID NO: 4.

There is provided, in another aspect of the present invention, a fusion peptide wherein a peptide having HTLV-I-specific CTL-inducing activity, consisting of the amino acid sequence shown in SEQ ID NO: 4, is bound to a marker protein and/or a peptide tag.

There is provided, in a further aspect of the present invention, a fusion peptide wherein a peptide having HTLV-1-specific CTL-inducing activity, consisting of an amino acid sequence wherein one amino acid is deleted, substituted or added in the amino acid sequence shown in SEQ ID NO: 4, is bound to a marker protein and/or a peptide tag.

In a further aspect of the present invention, there is provided a peptide fusion HLA-A24 protein wherein a peptide having HTLV-I specific CTL-inducing activity, consisting of the amino acid sequence shown in SEQ ID NO: 4, is bound to HLA-A24 protein.

In another aspect of the present invention, there is provided a peptide fusion HLA-A24 protein wherein a peptide having HTLV-I specific CTL-inducing activity, consisting of an amino acid sequence wherein one amino acid is deleted, substituted or added in the amino acid sequence shown in SEQ ID NO: 4, is bound to HLA-A24 protein.

There is provided, in a further aspect of the present invention, a tetramer of a peptide fusion HLA-A24 protein wherein a peptide having HTLV-1 specific CTL-inducing activity, consisting of the amino acid sequence shown in SEQ ID NO: 4, is bound to HLA-A24 protein.

There is provided, in another aspect of the present invention, a tetramer of a peptide fusion HLA-A24 protein wherein a peptide having HTLV-I specific CTL-inducing activity, consisting of an amino acid sequence wherein one amino acid is deleted, substituted or added in the amino acid sequence shown in SEQ ID NO: 4, is bound to HLA-A24 protein.

In another aspect of the present invention, there is provided a fusion protein wherein a peptide HLA-A24 fusion protein of the present invention as described herein or a tetramer of the present invention as described herein is bound to a marker protein and/or a peptide tag.

In another aspect, the present invention provides a DNA encoding the following peptide (a) or (b); (a) a peptide having HTLV-I-specific CTL-inducing activity consisting of the amino acid sequence shown in SEQ ID NO: 4, (b) a peptide having HTLV-I-specific CTL-inducing activity consisting of an amino acid sequence wherein one amino acid is deleted, substituted or added in the amino acid sequence shown in SEQ ID NO: 4.

There is provided, in another aspect of the present invention, a DNA consisting of the base sequence shown in SEQ ID NO: 8, or a complementary sequence thereof.

The present invention provides, in a further aspect, a HLA-A24-restricted Tax-epitope consisting of a peptide having HTLV-I-specific CTL-inducing activity consisting of the amino acid sequence shown in SEQ ID NO: 4.

There is provided, in another aspect of the present invention, a vaccine to induce immune response containing a peptide having HTLV-I-specific CTL-inducing activity consisting of the amino acid sequence shown in SEQ ID NO: 4, as active ingredient.

Further, there is provided in another aspect of the present invention, a vaccine to induce immune response containing a peptide having HTLV-I-specific CTL-inducing activity consisting of an amino acid sequence wherein one amino acid is deleted, substituted or added in the amino acid sequence shown in SEQ ID NO: 4, as active ingredient.

In another aspect of the present invention, there is provided a vaccine to induce immune response containing a vector capable of expressing DNA encoding the following peptide (a) or (b); (a) a peptide having HTLV-1-specific CTL-inducing activity consisting of the amino acid sequence shown in SEQ ID NO: 4, (b) a peptide having HTLV-1-specific CTL-inducing activity consisting of an amino acid sequence wherein one amino acid is deleted, substituted or added in the amino acid sequence shown in SEQ ID NO: 4, as active ingredient.

In a further aspect of the present invention, there is provided a vaccine to induce immune response containing a vector capable of expressing DNA consisting of the base sequence shown in SEQ ID NO: 8, or a complementary sequence thereof, as active ingredient.

The present invention provides, in a further aspect, a medical composition containing a vaccine to induce immune response containing a peptide having HTLV-I-specific CTL-inducing activity consisting of the amino acid sequence shown in SEQ ID NO: 4, as active ingredient.

The present invention provides, in another aspect, a medical composition containing a vaccine to induce immune response containing a peptide having HTLV-I-specific CTL-inducing activity consisting of an amino acid sequence wherein one amino acid is deleted, substituted or added in the amino acid sequence shown in SEQ ID NO: 4, as active ingredient.

The present invention provides, in a further aspect, a medical composition containing a vaccine to induce immune response containing a vector capable of expressing DNA encoding the following peptide (a) or (b); (a) a peptide having HTLV-I-specific CTL-inducing activity consisting of the amino acid sequence shown in SEQ ID NO: 4, (b) a peptide having HTLV-I-specific CTL-inducing activity consisting of an amino acid sequence wherein one amino acid is deleted, substituted or added in the amino acid sequence shown in SEQ ID NO: 4, as active ingredient.

The present invention provides, in another aspect, a medical composition containing a vaccine to induce immune response containing a vector capable of expressing DNA consisting of the base sequence shown in SEQ ID NO: 8, or a complementary sequence thereof, as active ingredient.

In another aspect of the present invention, there is provided a diagnostic agent to examine immune function containing a peptide having HTLV-I-specific CTL-inducing activity consisting of the amino acid sequence shown in SEQ ID NO: 4, as active ingredient.

In another aspect of the present invention, there is provided a diagnostic agent to examine immune function containing a peptide having HTLV-I-specific CTL-inducing activity consisting of an amino acid sequence, wherein one amino acid is deleted, substituted or added in the amino acid sequence shown in SEQ ID NO: 4, as active ingredient.

There is provided, in another aspect of the present invention, a diagnostic agent to examine immune function containing a vector capable of expressing the DNA encoding the following peptide (a) or (b): (a) a peptide having HTLV-I-specific CTL-inducing activity consisting of the amino acid sequence shown in SEQ ID NO: 4, (b) a peptide having HTLV-1-specific CTL-inducing activity consisting of an amino acid sequence wherein one amino acid is deleted, substituted or added in the amino acid sequence shown in SEQ ID NO: 4, as active ingredient.

There is provided, in a. further aspect of the present invention, a diagnostic agent to examine immune function containing a vector capable of expressing the DNA consisting of the base sequence shown in SEQ ID NO: 8, or a complementary sequence thereof, as active ingredient.

In a further aspect of the present invention, there is provided a diagnostic agent to examine immune function containing a peptide HLA-A24 fusion protein wherein a peptide having HTLV-1 specific CTL-inducing activity consisting of the amino acid sequence shown in SEQ ID NO: 4 is bound to HLA-A24 protein, as active ingredient.

In another aspect of the present invention, there is provided a diagnostic agent to examine immune function containing a peptide HLA-A24 fusion protein wherein a peptide having HTLV-I specific CTL-inducing activity consisting of an amino acid sequence wherein one amino acid is deleted, substituted or added in the amino acid sequence shown in SEQ ID NO: 4 is bound to HLA-A24 protein, as active ingredient.

There is provided, in another embodiment of the present invention, a diagnostic agent to examine immune function containing a tetramer of the peptide HLA-A24 fusion protein wherein the peptide having HTLV-1 specific CTL-inducing activity consisting of the amino acid sequence shown in SEQ ID NO: 4 is bound to HLA-A24 protein, as active ingredient.

There is provided, in another embodiment of the present invention, a diagnostic agent to examine immune function containing a tetramer of the peptide HLA-A24 fusion protein wherein the peptide having HTLV-I specific CTL-inducing activity consisting of an amino acid sequence wherein one amino acid is deleted, substituted or added in the amino acid sequence shown in SEQ ID NO: 4 is bound to HLA-A24 protein, as active ingredient.

In another embodiment of the present invention, there is provided a diagnostic agent of HTLV-I tumor containing the DNA encoding the following peptide (a) or (b); (a) a peptide having HTLV-I-specific CTL-inducing activity consisting of the amino acid sequence shown in SEQ ID NO: 4, (b) a peptide having HTLV-I-specific CTL-inducing activity consisting of an amino acid sequence wherein one amino acid is deleted, substituted or added in the amino acid sequence shown in SEQ ID NO: 4, as active ingredient.

In a further embodiment of the present invention, there is provided a diagnostic agent of HTLV-1 tumor containing the DNA consisting of the base sequence shown in SEQ ID NO: 8, or a complementary sequence thereof, as active ingredient.

There is provided, in another aspect of the present invention, an antibody binding specifically to the peptide having HTLV-I-specific CTL-inducing activity consisting of the amino acid sequence shown in SEQ ID NO: 4.

There is provided, in a further aspect of the present invention, an antibody binding specifically to the peptide having HTLV-I-specific CTL-inducing activity consisting of an amino acid sequence wherein one amino acid is deleted, substituted or added in the amino acid sequence shown in SEQ ID NO: 4.

There is provided, in a further aspect of the present invention, a diagnostic agent of HTLV-I tumor containing the antibody binding specifically to the peptide having HTLV-I-specific CTL-inducing activity consisting of the amino acid sequence shown in SEQ ID NO: 4, as active ingredient.

Further, there is provided in another aspect of the present invention, a diagnostic agent of HTLV-I tumor containing the antibody binding specifically to the peptide having HTLV-I-specific CTL-inducing activity consisting of an amino acid sequence wherein one amino acid is deleted, substituted or added in the amino acid sequence shown in SEQ ID NO: 4, as active ingredient.

In another aspect, the present invention provides an expression vector capable of expressing the peptide having HTLV-I-specific CTL-inducing activity consisting of the amino acid sequence shown in SEQ ID NO: 4.

In another aspect, the present invention provides an expression vector capable of expressing the peptide having HTLV-I-specific CTL-inducing activity consisting of an amino acid sequence wherein one amino acid is deleted, substituted or added in the amino acid sequence shown in SEQ ID NO: 4.

There is provided, in another aspect of the present invention, a host cell comprising an expression system capable of expressing the peptide having HTLV-I-specific CTL-inducing activity consisting of the amino acid sequence shown in SEQ ID NO: 4.

There is provided, in a further aspect of the present invention, a host cell comprising an expression system capable of expressing the peptide having HTLV-I-specific CTL-inducing activity consisting of an amino acid sequence wherein one amino acid is deleted, substituted or added in the amino acid sequence shown in SEQ ID NO: 4.

In addition, there is provided in another aspect of the present invention an expression vector capable of expressing the peptide HLA-A24 fusion protein wherein the peptide having HTLV-I-specific CTL-inducing activity consisting of the amino acid sequence shown in SEQ ID NO: 4 is bound to HLA-A24 protein.

In addition, there is provided in a further aspect of the present invention an expression vector capable of expressing the peptide HLA-A24 fusion protein wherein the peptide having HTLV-I-specific CTL-inducing activity consisting of an amino acid sequence wherein one amino acid is deleted, substituted or added in the amino acid sequence shown in SEQ ID NO: 4 is bound to HLA-A24 protein.

In another aspect, the present invention provides a host cell comprising an expression system capable of expressing the peptide HLA-A24 fusion protein wherein the peptide having HTLV-I-specific CTL-inducing activity consisting of the amino acid sequence shown in SEQ ID NO: 4 is bound to HLA-A24 protein.

In another aspect, the present invention provides a host cell comprising an expression system capable of expressing the peptide HLA-A24 fusion protein wherein the peptide having HTLV-I-specific CTL-inducing activity consisting of an amino acid sequence wherein one amino acid is deleted, substituted or added in the amino acid sequence shown in SEQ ID NO: 4 is bound to HLA-A24 protein.

The present invention provides, in a further aspect, a peptide having HTLV-I-specific CTL-inducing activity consisting of the amino acid sequence shown in SEQ ID NO: 4 for use in a method for inducing HTLV-I recognizing CTL, the method comprising using the peptide to stimulate PBMC of an HLA-A24 positive ATL patient.

The present invention provides, in another aspect, a peptide having HTLV-I-specific CTL-inducing activity consisting of an amino acid sequence wherein one amino acid is deleted, substituted or added in the amino acid sequence shown in SEQ ID NO: 4 for use in a method for inducing HTLV-I recognizing CTL, the method comprising using the peptide to stimulate PBMC of an HLA-A24 positive ATL patient.

In another aspect, the present invention provides a vector capable of expressing the DNA encoding the following peptide (a) or (b); (a) a peptide having HTLV-I-specific CTL-inducing activity consisting of the amino acid sequence shown in SEQ ID NO: 4, (b) a peptide having HTLV-I-specific CTL-inducing activity consisting of an amino acid sequence wherein one amino acid is deleted, substituted or added in the amino acid sequence shown in SEQ ID NO: 4, for use in a method for inducing HTLV-I recognizing CTL, the method comprising using the vector to stimulate PBMC of an HLA-A24 positive ATL patient.

In another aspect, the present invention provides a vector capable of expressing the DNA consisting of the base sequence shown in SEQ ID NO: 8, or a complementary sequence thereof, for use in a method for inducing HTLV-I recognizing CTL, the method comprising using the vector to stimulate PBMC of an HLA-A24 positive ATL patient.

There is described herein a peptide having HTLV-I-specific CTL-inducing activity comprised of an amino acid sequence shown in SEQ ID NO: 3 or 4 ("1"); a peptide having HTLV-I-specific CTL-inducing activity comprised of an amino acid sequence wherein one or a few amino acids are deleted, substituted or added in an amino acid sequence shown in SEQ ID NO: 3 or 4 ("2"); a fusion peptide wherein the peptide having HTLV-I-specific CTL-inducing activity according to "1" or "2" is bound to a marker protein and/or a peptide tag ("3"); a protein-peptide binding body wherein HLA-A24 and the peptide having HTLV-I-specific CTL-inducing activity according to "1" or "2" are bound ("4"): a tetramer of protein-peptide binding body wherein HLA-A24 and the peptide having HTLV-I-specific CTL-inducing activity according to "1" or "2" are bound ("5"): a fusion protein wherein the protein-peptide binding body according to "4" or the tetramer of protein-peptide binding body according to "5" is bound to a marker protein and/or a peptide tag (*6*): or a DNA encoding the following peptide (a) or (b); (a) a peptide having HTLV-I-specific CTL-inducing activity comprised of an amino acid sequence shown in SEQ ID NO: 3 or 4, (b) a peptide having HTLV-I-specific CTL-inducing activity comprised of an amino acid sequence wherein one or a few amino acids are deleted, substituted or added in an amino acid sequence shown in SEQ ID NO: 3 or 4 (*7*): a DNA comprised of a base sequence shown in SEQ ID NO.: 7 or 8, or a complementary sequence thereof (*8*): a DNA that hybridizes with the DNA according to *8* under a stringent condition and that encodes a peptide having HTLV-I- specific CTL-inducing activity ("9").

Moreover, there is described herein a HLA-A24-restricted Tax-epitope comprised of a peptide having HTLV-I-speciflc CTL-inducing activity having an amino acid sequence shown in SEQ ID NO: 4 (*10*): a vaccine to induce immune response containing a peptide having HTLV-I-specific CTL-inducing activity comprised of an amino acid sequence shown in SEa ID NO: 3 or 4, as active ingredient ("11"); a vaccine to induce immune response containing a peptide having HTLV-I-specific CTL-inducing activity comprised of an amino acid sequence as active ingredient, wherein one or a few amino acids are deleted, substituted or added in an amino acid sequence shown in SEQ ID NO: 3 or 4 ("12"); a vaccine to induce immune response containing a vector capable of expressing the DNA according to any one of "7" to "9", as active ingredient ("13"); and further, the vaccine to induce immune response according to any one of "8" to "10", comprising an adjuvant which enhances HTLV-I-specific CTL-inducing activity ("14"); a medical composition containing the vaccine to induce immune response according to any one of "11" to "14", as active ingredient ("15"): a diagnostic agent to examine immune function containing a peptide having HTLV-I-specific CTL-inducing activity comprised of an amino acid sequence shown in SEQ ID NO: 3 or 4, as active ingredient ("16"): a diagnostic agent to examine immune function containing a peptide having HTLV-I-specific CTL-inducing activity comprised of an amino acid sequence wherein one or a few amino acids are deleted, substituted or added in an amino acid sequence shown in SEQ ID NO: 3 or 4, as active ingredient ("17").

Further, there is described herein a diagnostic agent to examine immune function containing a vector capable of expressing the DNA according to any one of "7" to "9", as active ingredient ("18") : a diagnostic agent to examine immune function containing a protein-peptide binding body wherein HLA-A24 and the peptide having HTLV-1-specific CTL-inducing activity according to *1* or *2* are bound, as active ingredient ("19"); a diagnostic agent to examine immune function containing a tetramer of protein-peptide binding body wherein HLA-A24 and the peptide having HTLV-I-specific CTL-activity according to *1* or *2* are bound, as active ingredient ("20"); a diagnostic agent of HTLV-I tumor containing the DNA according to any one of *7* to *9* as active ingredient (*21*): an antibody binding specifically to the peptide having HTLV-I-specific CTL-inducing activity according to "1" or "2" ("22"): the antibody according to "22", wherein the antibody is a monoclonal antibody ("23") ; a diagnostic agent of HTLV-I tumor containing the antibody binding specifically to the peptide having HTLV-I-specific CTL-inducing activity according to "22" or "23", as active ingredient ("24"); an expression vector capable of expressing the peptide having HTLV-I-specific CTL-inducing activity according to "1" or "2" ("25"); or a host cell comprising an expression system capable of expressing the peptide having HTLV-1-specific CTL-inducing activity according to "1" or "2" ("26"); the expression vector capable of expressing a binding body of HLA-A24 and the peptide having HTLV-I-specific CTL-inducing activity according to *1* or *2* ("27"); a host cell comprised of an expression system capable of expressing a binding body of HLA-A24 and the peptide having HTLV-I-specific CTL-inducing activity according to *1* or *2* ("28"): a method for inducing HTLV-I recognizing CTL comprising the steps of: using HTLV-I infected T cells originating from pre-HSCT ATL patient, and to stimulate PBMC of the same ATL patient after HSCT. originating from HLA-identical donor ("29"): a method for inducing HTLV-I recognizing CTL comprising the steps of using the peptide having HTLV-I-specific CTL-inducing activity according to *1* or *2* and stimulating PMBC of HLA-A24 positive ATL patient ("30") ; and a method for inducing HTLV-I recognizing CTL comprising using a vector capable of expressing the DNA according to any of "7" to "9" and stimulating PMBC of HLA-A24 positive ATL patient ("31").
(1) The dominant epitope of CTL restricted by HLA-A24 that 60% or more of the Japanese have, has been found. By using the peptide of the present epitope site for examination of immune response to HTLV-I, it is possible to cover most of the Japanese group.
(2) Presently, it is possible to estimate the epitope from amino acid anchor motif having affinity for each HLA. However, the host immune response to pathogens in the living body does not always correspond to this estimation. The epitopes identified herein are obtained from infected individuals, and are recognized with extremely strong selectivity compared to other epitopes.
(3) It is rare that HTLV-I-specific CTL is induced from ATL patients. From ATL cases completely remitted after stem cells transplantation, CTL to epitopes identified herein were selectively induced. This shows that the epitope was strongly expressed in the patient body and that the epitope is useful as vaccine antigen.

### Brief Description of Drawings

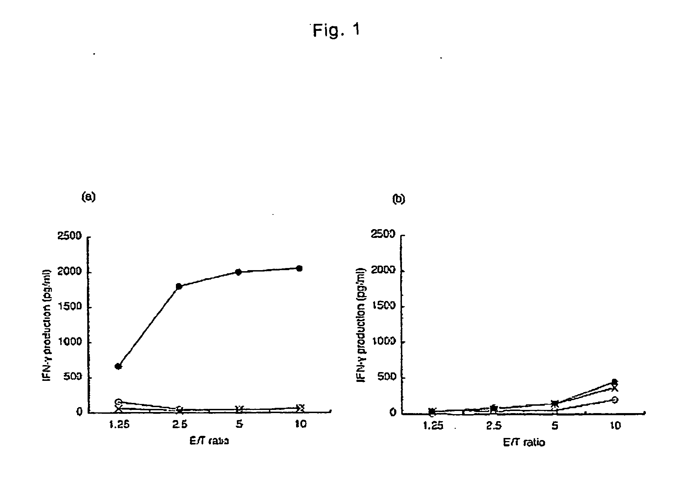

Fig. 1 is a picture showing induction of CTLs against ILT-#37 cells in PBMCs from post-HSCT patient #37 (Fig. 1a) and donor #36 (Fig. 1b). Various numbers of PBMCs were cultured for 19 days and stimulated twice with formalin-fixed ILT-#37 on day 0 and day 10. The PBMC were restimulated without (o) or with ILT-#37(●), or K562 (×), and the results of measurement with ELISA assay the levels of IFN-γ in the supernatants following 18 hours of incubation are shown in the figure. Values represent the mean of duplicate assays.

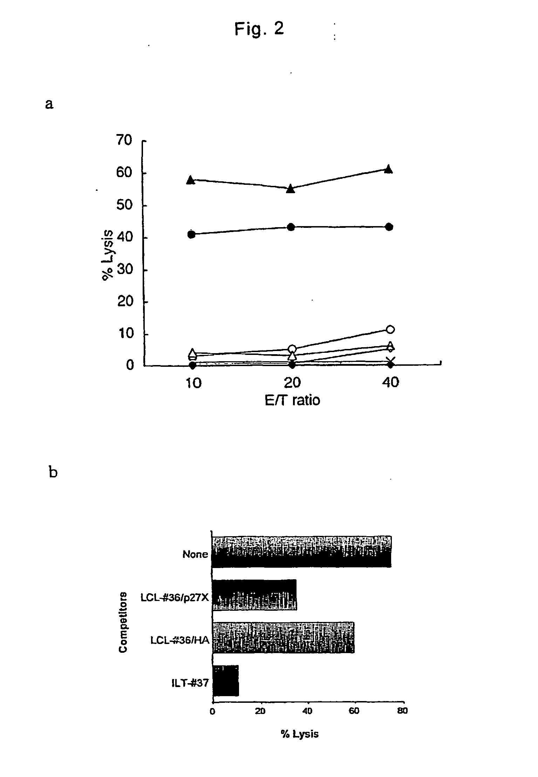

Fig. 2 is a picture showing HTLV-I Tax-specificity and MHC class I-restriction of CTLs induced from post-HSCT patient #37.
   a. PBMC culture of post-HSCT patient #37 was stimulated 4 times with formalin-fixed ILT-#37 and the cytotoxicity was examined by performing a 6-hour ⁵¹-Cr-release assay. The target cells used were HLA-identical ILT-#37(●), LCL-#36(○), and PHA-activated PBMC of pre-HSCT patient #37(×), HLA-A2 and B-46-matched TCL-Kan (▲) and LCL-Kan (Δ), and HLA-mismatched ILT-As-2 (**◆**) and LCL-As (◇). Closed symbols represent HTLV-I-infected cells while open symbols represent EBV-infected cells. Values represent the mean of specific lysis of triplicate assays.
   b. Blocking of ILT-#37 specific cytotoxicity by competitor cells expressing HTLV-I Tax is shown. PBMC cultures from post-HSCT patient #37 were stimulated 5 times with formalin-fixed ILT-#37 cells beforehand, and were subjected to ⁵¹Cr-release assay against radio-labeled ILT-#37 at an effector-to-target cells ratio of 30 to 1. The assay was performed in the presence of unlabeled LCL-#36 cells infected with rvv expressing HTLV-I pX gene products (LCL-#36/p27X) or unlabeled LCL-#36 cells infected with control rvv (LCL-#36/HA) or ILT-#37 cells, at a competitor-to-target cells ratio of 30 to 1.

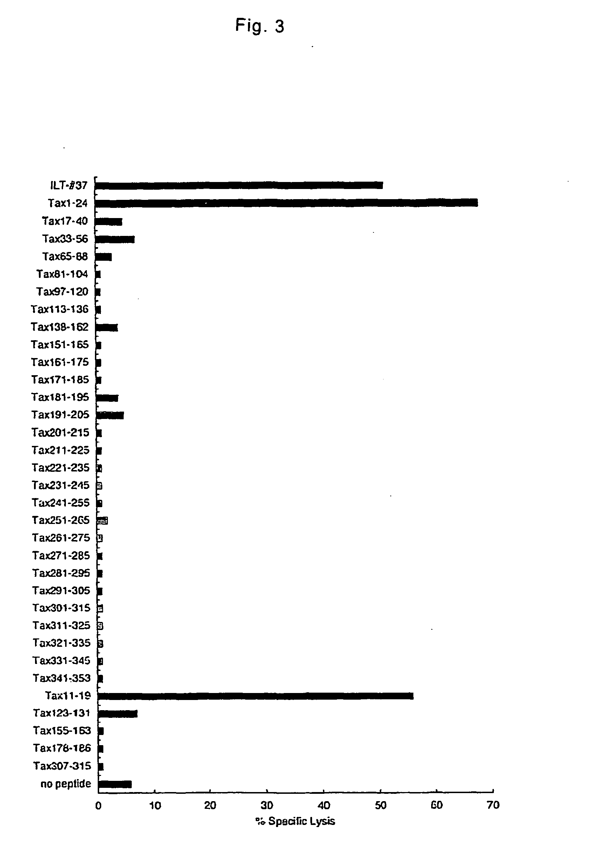

Fig. 3 is a picture showing the result of mapping of HLA-A2-restricted Tax epitopes of HTLV-I recognized by CTL induced frompost-HSCT patient #37 (case 1) . LCL-#36 cells were pulsed with 10 mM of 33 kinds of 9-24 mer synthetic oligopeptides corresponding to the Tax amino acid sequence, and their susceptibility to CTLs of post-HSCT patient #37 was measured by ⁵¹-Cr-release assays at an effector-to-target cells ratio of 10. Values represent the mean of specific lysis of triplicate assays.

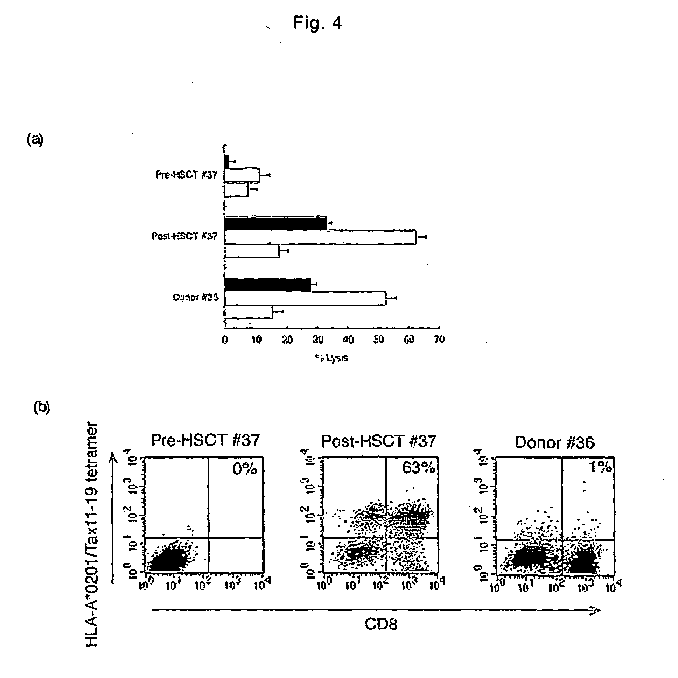

Fig. 4 is a picture showing the results of cytotoxic profiles in PBMC cultures from pre-HSCT patient #37, post-HSCT patient #37 and donor #36 induced by stimulation with ILT-#37 cells.
   a. Cytotoxicities of the PBMC from pre-HSCT patient #37 cultured for 40 days after two stimulations, and those from post-HSCT patient #37 and donor #36 cultured for 41 days after 3 stimulations were examined at an effector-to-target cells ratio of 30. Cytotoxicities against ILT-#37 (filled bars), TLC-Kan (hatched bars), and LCL-Kan (open bars) are shown, respectively. Values represent the mean of triplicate assays.
   b. Results of flow cytometric analysis of HLA-A*0201/Tax11-19 tetramer-binding CD8⁺ T cells in PBMC stimulated with ILT-#37 are shown. The PBMC cultures from post-HSCT patient #37 and donor #36 cultured for 46 days were used, while those from pre-HSCT patient #37 were used after 36 days of culture, because they failed to grow long-term. Tetramer specificity was confirmed by staining HTLV-I Taxll-19-specific cell line, Tc-Myj (Int. Immunol. 3, 761-767, 1991). Numbers in upper right corners indicate percentages of PBMC bound to the tetramer. A total of 100, 000 events is shown in each case.

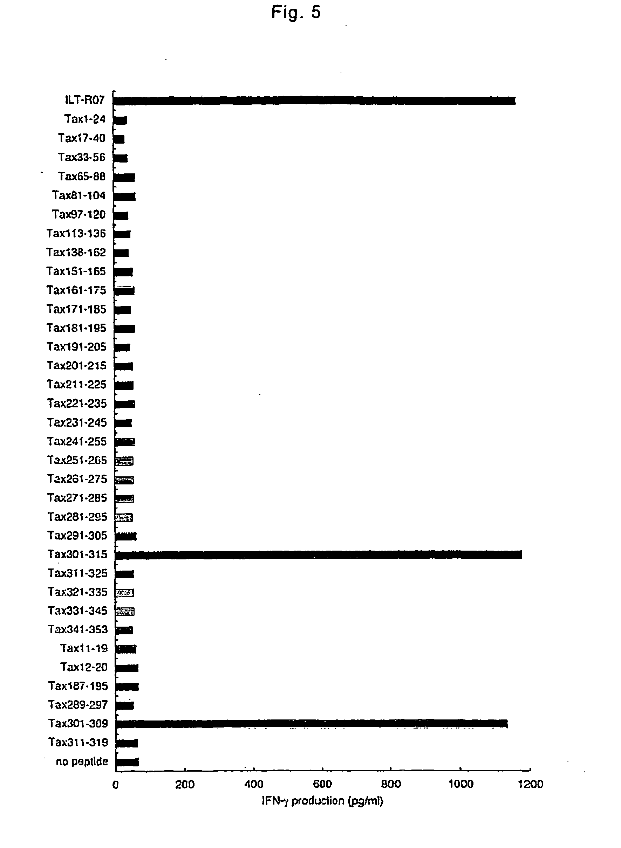

Fig. 5 is a picture showing the result of mapping of HTLV-I HLA-A24-restricted Tax epitopes recognized by CTL induced from post-HSCT patient R07 (case 2). PBMCs containing abundantly CD8⁺ cells were stimulated 3 times with formalin-fixed ILT-R07 cells, cultured for 32 days, mixed with TOK which is HLA-A24+ EBV transformed B cell line pulsed with a series of 33 synthetic oligopeptides of Tax, at an effector-to-target ratio of 8. Following 18 hours of incubation, IFN-γ in the supernatant was measured by ELISA assay. Values represent the mean of duplicate assays.

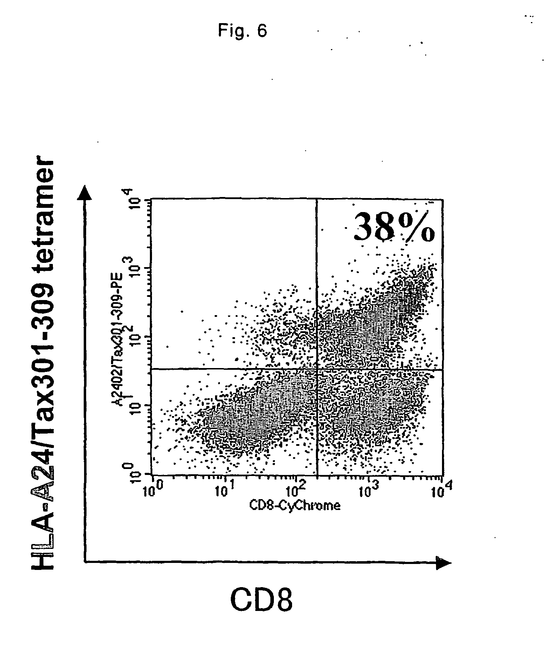

Fig. 6 is a picture showing the result of flow cytometry analysis of HLA-A*2402/Tax301-309 tetramer-binding CD8⁺ T cells in CTL induced from post-HSCT patient R07 (case 2). PBMCs cultured for 67 days from post-HSCT patient R07 were used. Numbers in upper right corners indicate percentages of PBMC bound to the tetramer. A total of 100,000 events were collected in each case.

As for the peptide having HTLV-I-specific CTL-inducing activity mentioned herein in other words a peptide that can induce CTL having specific anti-tumor effect to HTLV-I tumor, it is not particularly limited as long as it is a peptide comprised of an amino acid sequence shown in SEQ ID NO: 3 or 4, or a peptide having CTL-inducing activity specifically to HTLV-I, comprised of an amino acid sequence wherein one or a few amino acids are deleted, substituted or added in an amino acid sequence shown in SEQ ID NO: 3 or 4 (hereinafter, a peptide comprised of an amino acid sequence shown in SEQ ID NO: 3 or 4, or a peptide modified by one or a few amino acids being deleted, substituted or added in these amino acid sequences, may be referred together as "peptides mentioned herein "). Here, as for the level of "substitution, deletion or addition" of amino acids, or the site thereof, it is not particularly limited as long as the modified peptide is a material with the same effect having HTLV-I-specific CTL-inducing activity similar to the peptide comprised of amino acid sequences shown in SEQ ID NO: 3 or 4. The modification (mutation) of amino acid sequence can occur for example by mutation or modification after translation, while it may be modified artificially. In the description, all of the modified peptides having the above characteristics are encompassed, no matter the cause or means of modification/mutation.

The peptides mentioned herein can be generated by chemical or genetic engineering methods. The chemical methods include peptide synthesis methods by usual liquid phase and solid phase methods. The peptide synthesis methods include, more specifically, the stepwise elongation method comprising elongating the chain by binding the amino acids one by one sequentially according to the amino acid sequence information, and the fragment condensation method comprising synthesizing fragments comprised of a few amino acids beforehand, and reacting each fragment by coupling. Synthesis of the peptides comprised of amino acid sequence shown in SEQ ID NO: 3 can be performed by any one of these methods.

As for the condensation method applied to the above peptide synthesis can be performed according to various known methods. These methods include, for example, azide method, mixed acid anhydride method, DCC method, active ester method, oxidation-reduction process, DPPA (diphenyl phosphoryl azide) method, DCC + additives (1-hydroxybenzotriazole, N-hydroxysuccinamide, N-hydroxy-5-norbornene-2,3-dicarboxyimide, etc.), and woodward method. The solvents that can be used for these methods can be selected appropriately from those generally known to be used to such peptide condensation reaction. These solvents include dimethylformamide (DMF), dimethylsulfoxide (DMSO), hexaphosphoramide, dioxane, tetrahidrofuran (THF), ethyl acetate, and mixed solvent thereof.

Meanwhile, for the above peptide synthesis reaction, carboxyl group of amino acid or peptide that is not involved in the reaction, can be protected, for example, as lower alkylester, such as for example methyl ester, ethyl ester, tertiary butyl ester ; for example benzyl ester, p-methoxy benzyl ester, p-nitrobenzyl ester, p-nitrobenzylesteraralkylester and the like, generally by esterification. Moreover, an amino acid having a functional group on the side chain, for example hydroxyl group of Tyr, can be protected with acetyl group, benzyl group, benzyloxycarbonyl group, tertiary butyl group and the like, but such protection is not always necessary. Moreover, for example guanidine group of Arg can be protected by appropriate protecting group such as nitro group, tosyl group, 2-methoxybenzenesulfonyl group, methylene-2-sulfonyl group, benzyloxycarbonyl group, isobornyloxycarbonyl group, adamantyloxycarbonyl group. Deprotection reaction of these protecting group in the amino acid, peptides and the peptides having the above protecting group and the peptides mentioned herein finally obtained can be performed according to usual methods, including catalytic reduction method or methods using liquid ammonia/sodium, hydrogen fluoride, hydrogen bromide, hydrochloric acid, trifluoroacetate, acetic acid, formic acid, methanesulfonic acid, and the like.

The peptides mentioned herein can be obtained by chemical synthesis as mentioned above, and also by common methods using genetic engineering method. The peptides mentioned herein thus obtained can be purified appropriately according to methods commonly used in the field of peptide chemistry, such as ion exchange resin, partition chromatography, gel chromatography, affinity chromatography, high performance liquid chromatography (HPLC) and counter current distribution method, according to usual methods.

As for the fusion peptides described herein, it is not particularly limited as long as the peptides mentioned herein are bound with the maker protein and/or peptide tag. As for the marker proteins, it is not particularly limited as long as it is a marker protein conventionally known, including alkaline phosphatase, Fc region of the antibody, HRP and GFP. Specific examples of peptide tag include epitope tag such as HA, FLAG, Myc; affinity tag such as GST, maltose-binding protein, byotinylated peptide and oligohistidine, which are conventionally known peptide tags. The fusion peptides can be prepared by common methods, and are useful for the purification of the peptides mentioned herein - using the affinity of Ni-NTA and His tag, for the detection of the peptides mentioned herein , the quantitation of antibodies against the peptides mentioned herein, or also as reagents for researches of the art.

As for the protein-peptide binding body described herein, it is not particularly limited as long as it is a binding body of HLA-A24 and the peptides mentioned herein, and those with a morphology capable to bind with CTL that can recognize the binding body such as binding body of, for example, HLA-A24 molecule and a peptide comprised of amino acid sequence shown in SEQ ID NO: 4 are preferable. Further, as for the tetramer of the protein-peptide binding body described herein, it is not particularly limited as long as it is a tetramer of the protein-peptide binding body wherein HLA-A24 and the peptides mentioned herein are bound, and a tetramer prepared with the above protein-peptide binding body with streptavidin as the nucleus can be exemplified. For example, it can be obtained by expressing the substrate of the enzyme Bir-A at the C-terminal of HLA-A24, and by mixing HLA-A24 biotinylated by Bir-A-dependent biotinilation method and phycoerythrin (PE) -labeled deglycosylated avidin at a rate of 4:1 (Altman, J. D., et al.: Science 274, 94-96, 1996). These protein -peptide binding body and tetramer thereof can be prepared by binding the peptides mentioned herein chemically synthesized with α-domain of HLA-A24 or β-2 microglobulin, prepared by a common method with genetic engineering using HLA-A24 gene (accession number AAB60651) or β-2 microglobulin gene (accession number NM_004048), in a refolding buffer in vitro (Garboczi et al. Proc. Natl. Acad. Sci. USA., 89: 3429-3433, 1992); or by coexpressing in the same host cell, the peptides mentioned herein and α-domain of HLA-A24 or β-2 microglobulin, prepared by a common method with genetic engineering using DNA encoding the peptides mentioned herein and HLA-A24 gene or β-2 microglobulin gene, respectively, and by binding these after purification.

As for the fusion protein described herein it is not particularly limited as long as the above protein-peptide binding body or the tetramer of the protein-peptide binding body is bound with maker protein and/or peptide tag. As for marker protein, it is not particularly limited as long as it is a marker protein conventionally known, and fluorochrome, alkaline phosphatase, Fc region of the antibody, HRP, GFP can be specifically exemplified. As for peptide tag, peptide tag that are conventionally known including epitope tag such as HA, FLAG and Myc, affinity tag such as GST, maltose-binding protein, biotinylated peptide, oligohistidine can be specifically exemplified. The fusion proteins can be prepared by common methods, and, are useful for the purification of protein-peptide binding body using the affinity of Ni-NTA and His tag, for the detection of CTL, the quantitation of antibodies against the peptides mentioned herein, or also as reagents for researches of the art.

As for the antibodies binding specifically to the peptides mentioned herein, immune-specific antibodies such as monoclonal antibody, polyclonal antibody, chimeric antibody, single-stranded antibody, humanized antibody can be specifically exemplified. These can be prepared by common methods by using the above peptides mentioned herein as antigen, and among these, monoclonal antibodies are preferable from its specificity. The antibodies binding specifically to the peptides mentioned herein such as monoclonal antibodies are not only useful for the diagnosis of HTLV-I tumor such as ATL, but also for revealing activation mechanism or molecular mechanism of HTLV-I-specific CTL-induction of the peptides mentioned herein.

The antibodies against the peptides mentioned herein are generated by administering the peptides mentioned herein complex of the peptides mentioned herein and protein having immunogenecity, the cells presenting the peptides mentioned herein to its membrane surface, to animals (preferably other than human) by using the traditional protocols. For example, for the preparation of monoclonal antibodies, any methods that generate antibodies from cultures of continuous cell system including hybridoma method (Nature 256 , 495-497, 1975); trioma method, human B cells hybridoma method (Immunology Today 4, 72, 1983), and EBV-hybridoma method (MONOCLONAL ANTIBODIES AND CANCER THERAPY, pp. 77-96, Alan R. Liss, Inc., 1985) can be used.

As for the DNAs mentioned herein, it is not particularly limited as long as it is a DNA encoding the above peptides mentioned herein, a DNA comprised of a base sequence shown in SEQ ID NO:7 or 8 or its complimentary sequence, or a DNA that hybridizes with the DNA comprised of a base sequence shown in SEQ ID N0:7 or 8 or its, complimentary sequence under a stringent condition, and that encodes a peptide having HTLV-I-specif ic CTL-inducing activity (hereinafter, the above DNAs mentioned herein may be collectively referred to as -the DNAs mentioned herein "), As for the above condition to "hybridize under a stringent condition", for example, hybridization at 42°C, and washing treatment with a buffer containing 1 x SSC and 0.1% SDS at 42°C can be exemplified, and hybridization at 65°C and washing treatment with a buffer containing 0.1 x SSC and 0.1% SDS at 65°C can be more preferably exemplified. Meanwhile as for factors influencing the stringency of the hybridization, there are various factors other than the above temperature condition, and a person skilled in the art can realize a similar stringency to that of the above exemplified one by combining various factors. The DNAs mentioned herein can be used advantageously when preparing the peptides mentioned herein by using genetic engineering with common method, and the antisense chain of the DNAs mentioned herein is particularly useful as a diagnostic probe for HTLV-I tumor such as ATL.

As for the HLA-A24-restricted Tax epitope described herein it is not particularly limited as long as it is an epitope comprised of the peptide having HTLV-I-specific CTL-inducing activity having an amino acid sequence shown in SEQ ID NO: 4, that can induce CTL in vivo and in vitro. The peptides mentioned herein including the HLA-A24-restricted Tax epitope or the vectors capable of expressing the DNAs mentioned herein can be used as active ingredient of the vaccine for inducing immune response mentioned herein for cellular immunity or humoral immunity. The vaccine for inducing immune response mentioned herein can be used for treating HTLV-I tumor such as ATL.

Further, as for the vaccine for inducing immune response mentioned herein, those containing various adjuvants enhancing cellular or local immunity are more preferable. Those adjuvants include, for example, dendritic cells capable of inducing effectively peptide-specific CTL, ISS-ODN including CpG motif (Immunostimulatory DNA sequences-oligodeoxynucleotide; Nat. Med. 3, 849-854, 1997), QS21 stimulating cytotoxic T cells (commercially available from Quillaia saponaria, Cambridge Biotech, Worcester, MA), aluminium hydroxide, aluminium phosphate, aluminium oxide, oily emulsion, saponin, and vitamin E lysis. When using the adjuvants, they can be used as recombinant fusion proteins or recombinant fusion peptides prepared from DNA encoding sequently various bacterial components or toxins to become adjuvant, with the peptides mentioned herein,

Moreover, the medical compositions mentioned herein containing the above vaccine for inducing immune response as active components, can include pharmaceutically acceptable carriers or diluents, adjuvants or additives. As for the carriers or diluents, for example, stabilizer such as SPGA, carbohydrate such as sorbitol, mannitol, starch, sucrose, glucose, dextran; protein such as alubumin, casein; substances containing protein such as bovine serum or skim milk; buffer solution such as phosphate buffer, physiological saline and water can be specifically exemplified. As for the adjuvants, cytokine such as Interleukln-2 (IL-2), Interleukin-12 (IL-12), tumor necrosis factor a (THF-a) can be specifically exemplified. The additives include low molecular polypeptide (about 10 residues or less), protein, amino acid, carbohydrate including glucose or dextran, chelating agent such as EDTA, protein stabilizer, substances inhibiting or suppressing proliferation of microorganisms, but they are not limited to these examples.

Moreover, the medical compositions mentioned herein are preferable to be in a form that can be administered orally, intravenously, intraperitoneally, intranasally, intracutaneously, subcutaneously, or intramuscularly. The effective dose to administer can be determined appropriately by considering the types or compositions of medical agents or medical compositions, administration methods, the age or body weight of patients, etc. It is effective to administer the dose one or a few times per day. Moreover, when administering orally, it is generally administered in the form of formulation prepared by mixing with carrier for formulation. As for the carrier that can be used for formulation, substances used commonly in the field of formulation, and that do not react with the peptide mentioned herein are used. As for dosage form, tablets, capsules, granules, powder, syrup, suspension, suppository, ointment, cream, gel, adhesive preparation, inhalant, injection and the like can be specifically exemplified. These formulations are prepared according to common methods, and particularly, when it is in a form of liquid formulation, it can be diluted or suspended in water or other appropriate medium at the time of use. Moreover, tablets and granules can be coated by a known method. In case of injection, it is prepared by dissolving the peptides mentioned herein in water, while it can be dissolved in physiological saline or glucose solution if necessary, or add buffer agent or preservatives. Further, other components being valuable for therapy may be contained in these formulations.

Additionally, the peptides mentioned herein can be intake as functional food by mixing with various foods including baked cakes such as caramel, cookie, bread, cake, jelly, rice cracker; Japanese cakes such as bean jelly; breads/snacks such as cold dessert, chewing gum; noodles such as wheat noodle and buckwheat noodle; fish cakes such as steamed fish paste, ham, fish meat sausage; various drinks such as yoghurt,' yoghurt drink, juice, milk, soy milk, alcohols, coffee, tea, boiled tea, oolong tea, sport drink; seasonings such as soybean paste (miso), soybean sauce, dressing, mayonnaise, sweetener; various prepared foods such as soybean cake, arum root, other fish boiled in soy sauce, jaozi, croquette and salad, as food material preventing HTLV-1 infection and/or ameliorating symptoms of HTLV-I related diseases.

As for diagnostic agent to examine immune function, it is not particularly limited as long as it comprises as active ingredients the peptides mentioned herein, the vectors capable of expressing the DNAs mentioned herein the protein-peptide binding body wherein HLA-A24 and the peptides mentioned herein, are bound, or the tetramer of the protein-peptide binding body, and that can examine and diagnose immune function, particularly immune function to HTLV-I. However, generally it is preferable to use a labeled body of the peptides mentioned herein; a vector capable of expressing the DNAs mentioned herein wherein the expressed products are the labeled bodies, a labeled body of the protein-peptide binding body wherein HLA-A24 and the peptides mentioned herein are bound, or a labeled body of tetramer of the protein-peptide binding body. As for labeling substance used for making labeled body, radioactive isotopes can be used beside the above marker protein or peptide tag. The diagnosis to examine immune function by using the diagnostic agent to examine immune function can discriminate HTLV-I Tax specific-T cells by contacting the diagnostic agent to examine immune function to peripheral blood leukocyte (lymphocyte) of the tested individual, and to bind T cells recognizing epitope in the peptides mentioned herein and the like. Among the diagnostic agents to examine immune function, as fluoro-labeled body such as PE of tetramer of the above protein-peptide binding body makes it possible to make the detect/quantify CTL by flow cytometry, it is particularly useful for determining the vaccine effect beside being useful as diagnostic agent to examine immune function. For example, it is possible to examine and make a diagnosis of immune function of the tested individuals, by separating mononuclear fraction from heparin peripheral blood sample, double-staining with PE-labeled tetramer (tetramer of protein-peptide binding body) and activated marker antibody such as CD8 antibody labeled with FITC or Cy5, and by calculating the number of CD8 and tetramer positive cells with flowcytometer. Further, it happens often that the number of tetramer positive cells are very few in fresh blood samples, it is possible to perform similar staining analysis not only to fresh blood samples, but after stimulating once with the peptides mentioned herein or cells expressing thereof and conducting culture for a few days to a week.

As for the expression vectors described herein, it is not particularly limited as long as it can express the peptides mentioned herein, or a binding body of HLA-A24 (a domain and/or β-2 microglobulin) and the peptides mentioned herein. The expression system used is not limited as long as it is an expression system capable of expressing the above peptides mentioned herein in the cells, and include, expression systems derived from chromosome, episome and virus, for example, vectors derived from bacterial plasmid, yeast plasmid, papovavirus such as SV40, vaccinia virus, adenovirus, fowl poxvirus, pseudorabies virus, retrovirus; vectors derived from bacteriophage, transposon, or from combination thereof, for example those derived from genetic elements of plamid and bacteriophage such as cosmids and phagemids. Among these, viral vectors are preferable. These expression systems can include a regulatory sequence that can not only induce expression but also to control the expression. Moreover, a series of expression vector that can translate by changing the open reading frame can be used advantageously. The expression vector described herein is useful as active ingredient in the vaccine for inducing immune response.

As for the host cells described herein, it is not particularly limited as long as it is a cell comprising the expresion system capable of expressing the peptides mentioned herein and the binding body of HLA-A24 (a domain and/or β-2 microglobulin) and the peptides mentioned herein.

As for the host cells used, bacterial prokaryotic cells such as B. Coli, streptomyces, Bacillus subtilis, Streptococcus and Staphylococcus: eukaryotic cells such as yeast and Aspergillus; insect cells such as Drosophila S2 and Spodoptera Sf9; plant and animal cells such as L cells, CHO cells. COS cells, HeLa cells, C127 cells and BALB/c3T3 cells (including mutant strain lacking dihydrofolate reductase or thymidine kinase), BHK21 cells, HEK293 cells. Bowes melanoma cells and oocytes can be exemplified. Moreover, as for the method for introducing expression systems capable of expressing the peptides mentioned herein to the host cells, various methods described in standard laboratory manuals such as Davis et al. (BASIC METHODS IN MOLECULAR BIOLOGY, 1986) and Sambrook et al. (MOLECULAR CLONING: A LABORATORY MANUAL. 2nd Ed., Cold Spring Harbor Laboratory Press, Cold Spring Harbor, N.Y., 1989) can be used, including calcium phosphate transfection, DEAE-dextran mediated transfection, transvection, microinjection, cationic lipid mediated transfection, electroporation, transduction, scrape loading, ballistic introduction or infection. These host cells described herein are useful for revealing the activating mechanism or molecular mechanism of HTLV-I-specific-CTL induction of the peptides mentioned herein.

As for the method for inducing CTL recognizing HTLV-I described herein, it is not particularly limited as long as it is a method for inducing CTL recognizing HTLV-I by stimulating the PBMC of HLA-A24 positive ATL patient, in vitro, in vivo or ex vivo: for example, a method for inducing CTL recognizing HTLV-I by using HTLV-I infected T cells derived from pre-HSCT ATL patient, and by stimulating PBMC of the same patient after HSCT from a donor of identical HLA, that is, HLA-A24 type, in vitro, in vivo or ex vivo; a method for inducing HTLV-I recognizing CTL by using the peptides mentioned herein, and by stimulating the PBMC of HLA-A24 positive ATL patient in vitro, in vivo or ex vivo; or a method wherein by using the vectors capable of expressing the DNAs mentioned herein, for example the peptide is expressed in antigen-presenting cells in PBMC by genetic engineering, etc. The HTLV-I recognizing CTL obtained by these induction methods can be used for treating HTLV-I tumor such as ATL as adoptive immunotherapy, and is also useful for revealing activation mechanism and molecular mechanism of HTLV-I-specific CTL induction.

### (Examples)

The present invention will be explained further in detail in the following, but the scope of this invention will be not limited to these examples.

### A [Methods and Materials]

### A-1 (Recipient/donor pairs and blood samples)

Two acute type ATL patients, #37 (case 1) and R07 (case 2), and their corresponding HLA-identical sibling donors, #36 and D07, donated peripheral blood samples. Although the patient #37 had transient recurrence of ATL in four weeks of HSCT, both cases obtained complete remission within two months after HSCT. Heparinized blood was collected on day -12 and day +183 from patient #37 and on day -30 and day +255 from patient R07. Their peripheral blood mononuclear cells (PBMC) isolated on a Ficoll-Hypaque PLUS (Amersham Bioscience, Piscataway, New Jersey) gradient were partially stored in liquid nitrogen until use.

### A-2 (Cell lines)

ILT-#37 and ILT-R07 which are HTLV-1-infected T-cell lines originating from pre-HSCT patients #37 and R07 were established as follows: CD8⁺ cells were depleted from PBMCs using a Dynabeads M450-CD8 (Dynal, Oslo, Norway), and then the resultant was stimulated with 1 µg/ml of phytohemagglutinin (PHA)-P (SIGMA, St. Louis, Missouri), and then maintained in RPMI-1640 (GIBCO-Invitrogen Grand Island, New York) medium containing 10% heat-inactivated fetal calf serum (FCS) (SIGMA), 10 U/ml recombinant human IL-2 (Shionogi, Osaka, Japan) or 10 ng/ml recombinant human IL-15 (SIGMA) at 37°C with 5% CO₂ for over 2 months. An Epstein-Barr virus (EBV)-transformed B-cell line, LCL-#36, was established from CD19⁺ PBMC of donor #36 infected with EBV-containing supernatant of B95-8 cells (J. Immunol. 124, 1045-1049, 1980) in vitro. HTLV-I-infected T-cell lines, TCL-Kan (Int. J. Cancer 34, 221-228, 1984) and ILT-As-2 (Int. Immuno. 3, 761-767, 1991), EBV-transformed B-cell lines LCL-Kan, LCL-As, andTOK (J. Virol. 66, 2928-2933, 1992), and an erythroblastoid cell line K562 (Blood 45, 321-334, 1975) were also used.

### A-3 (Induction of HTLV-I-specific CTLs)

One million whole cell- or CD8⁺ cell-enriched PBMCs from post-HSCT patients #37 and R07 were stimulated with 1 µg/ml PHA-P, then mixed with the same number of ILT-#37 or ILT-R07 cells pre-treated with 1% formaldehyde/PBS. These T-cells were maintained in AIM-V™ medium (GIBCO-Invitrogen) supplemented with 10% FCS and 100 U/ml recombinant human IL-2 with periodic stimulation with respective ILT cells at 10- to 14-day intervals.

### A-4 (Synthetic peptides)

The present inventors prepared a total of 38 peptides (9 to 24-mer) to cover the entire sequence of the HTLV-I Tax protein. Some of the peptides were synthesized as described previously (J. Natl. Cancer Inst. 93, 1775-1783, 2001; J. Virol. 66, 2928-2933, 1992) and all other 9-mer peptides were purchased from Hokudo Co. (Hokkaido, Japan). To identify potential HLA-A2- or A24- binding peptides within HTLV-I Tax, a computer-based program, BIMAS (http=//bimas.dcrt.nih.gov/molbil/hl.a#bind/), was employed as described previously (J. Immunol. 152, 163-175, 1994; J. Immunol. 152, 3913-3924, 1994).

### A-5 (CTL assay)

Cytotoxic activities were measured by 6-hour ⁵¹Cr-release assay at various effector-to-target (E/T) cell ratios as described previously (J. Natl. Cancer Inst. 93, 1775-1783, 2001; J. Virol. 76, 7010-7019, 2002). Specific cytotoxicity was calculated as ([experimental ⁵¹Cr release - spontaneous ⁵¹Cr release] / [maximum ⁵¹Cr release - spontaneous ⁵¹Cr release] x 100%). IFN-γ production by the effector cells was measured by ELISA (Human IFN-γ ELISA Kit; Endogen, Woburn, Massachusetts) following 18 hours of incubation with target cells at various E/T ratios in duplicate.

### A-6 (Recombinant vaccinia viruses)

Recombinant vaccinia virus (rvv) WR-p27^{x} containing HTLV-1 pX genes and WR-HA without the HTLV-1 gene were kindly provided by Dr. Hisatoshi Shida (Hokkaido University, Japan) (Cell 55 , 197-209, 1988) and used at a multiplicity of infection (MOI) of 50 as described previously (Int. Immunol. 3, 761-767, 1991; J. Natl. Cancer Inst. 93, 1775-1783, 2001).

### A-7 (ELISPOT assays)

IFN-γ producing antigen-specific T cells were counted using IFN-γ-specific ELISPOT assays as previously described (J. Immunol. Methods 191, 131-142, 1996; J. Exp. Med. 186, 859-865, 1997). Briefly, PBMCs were placed in a 96-well PVDF plate (ELISIP10SSP, Millipore, Bedford, Massachusetts) pre-coated with anti-IFN-γ mAb, 1-D1K (MABTECH, Nacka, Sweden) in triplicate at 1 × 10⁵ cells/well with 5 × 10⁴ cells/well of stimulator cells or 10 µg/ml peptides overnight at 37°C. The next day, cells were removed and the wells were incubated with biotinylated anti-IFN-γ mAb, 7-B6-1 biotin (MABTECH) for 2 hours then with streptavidin-alkaline phosphatase (MABTECH) for an additional 1 hour. After 10-min reaction with BICP/NBT alkaline phosphatase substrate (SIGMA), colored spots on the dried membranes were counted using a KS-ELISPOT microscopy system (Carl Zeiss, Jena, Germany).

### A-8 (Tetramer staining)

Phycoerythrin (PE)-conjugated HLA-A*0201/Taxll-19 (LLFGYPVYV; SEQ ID NO: 1) tetramer and phycoerythrin (PE)-conjugated HLA-A*2402/Tax301-309 (SFHSLHLLF; SEQ ID NO: 4) tetramer were provided by the NIAID Tetramer Facility, Emory Univ. Vaccine Center at Yerks (Atlanta, Georgia), where the synthesis was committed. Lymphocytes were stained for 30 min with Cy-Chrome™-conjugated anti-CD8 mAb (BD Pharmingen) and subsequently with tetramer for an additional 60 min at 4°C, then subjected to two-color analysis on a FACSCalibur using CellQuest software (Beckton Deckinson) (J. Immunol. 162, 1765-1771, 1999).

### B [Results]

### B-1 (Induction of CTL from post-HSCT recipient reacting with pre-HSCT HTLV-I-infected cells)

In order to examine immune response of the post-HSCT-recipients to hematopoietic cells of pre-HSCT-recipients origin, the present inventors established T cell lines, ILT-#37 and ILT-R07, from pre-HSCT patients, #37 and R07, respectively, by maintaining PHA-stimulated PBMC for longer than 2 months in the presence of IL-2 or IL-15. Both of these cell lines were positive for CD4 and HTLV-I antigens such as HTLV-I Tax and p19.

T-cell response in the PBMC of post-HSCT patient #37 to ILT-#37 cells was examined at +183 days of HSCT, when hematopoietic cells had been completely replaced by those of donor origin. Since donor #36 was an HTLV-I-carrier, T-cell response of donor #36 to ILT-#37 was also examined. The PBMC from post-HSCT patient #37 and donor #36 stimulated in vitro with formaldehyde-treated ILT-#37 twice in the presence of IL-2 were examined for interferon gamma (INF-γ)-producing ability against ILT-#37 and K562 cells at 19 days after initiation of culture. IFN-γ was produced in both cultures from post-HSCT #37 and donor #36 against ILT-#37, but not against K562 cells following overnight incubation (Fig. 1). The magnitude of the IFN-γ-producing response was significantly greater in the culture of post-HSCT #37 than in that of donor #36. Cell proliferation was also faster in the culture for post-HSCT patient #37 than in that for donor #36. The present inventors confirmed that the effector cells from post HSCT patient #37 were mostly CD8⁺ cytotoxic T lymphocytes (CTL) capable of killing ILT-#37 cells by ⁵¹Cr-release assay.

### B-2 (HTLV-I-specificity of the CTL line induced from post-HSCT patient)

The specificity of the CTL originating from post-HSCT #37 in response to stimulation with ILT-#37 cells was then assessed. Significant levels of cytotoxicity were observed against ILT-#37 but not against PHA-stimulated PBMC of pre-HSCT patient #37 (Fig. 2a). This indicated that the main target antigens of the CTL were those preferably expressed on. ILT-#37 but not on PHA-stimulated PBMC, although both of these target cells originated from pre-HSCT patient #37. Furthermore, the CTL efficiently killed allogeneic HTLV-I infected TCL-Kan cells sharing HLA-A2 and B46 but not HLA-mismatched HTLV-I-infected ILT-As-2, EBV-infected LCL-#36 originating from HLA-identical donor #36, LCL-Kan nor LCL-As cells. These results strongly indicated that the CTL line established from post-HSCT patient #37 (CTL line of post-HSCT patient #37) in response to ILT-#37 were specific for HTLV-I antigens.

The cytotoxicity of CTL line of post-HSCT patient #37 for radiolabeled ILT-#37 was significantly, but partially inhibited by unlabeled LCL-#36 infected with vaccinia recombinants expressing HTLV-I pX gene products including Tax (LCL-#36/p27X) (Fig. 2b), indicating that the CTL line of post-HSCT patient #37 included a large number of HTLV-I Tax-specific CD8* CTL capable of lysing ILT-#37 cells.

The present inventors further examined the epitopes in HTLV-I Tax recognized by CTL line of post-HSCT patient #37 with a panel of 15 to 24-mer oligopeptides corresponding to the Tax amino acid sequence and five 9-mer peptides that were the most probable HLA-A2-restricted Tax epitopes as predicted by a computer program based on the anchor motifs. LCL-#36 cells pulsed with the oligopeptides Taxl-24 (MAHFPGFGQSLLFGYPVYVFGDCV; SEQ ID NO: 2) and Taxll-19 (LLFGYPVYV; SEQ ID NO: 1) were selectively killed by CTL line of post-HSCT patient #37, indicating that the major population of HTLV-I Tax-specific CTL in the culture of CTL line of post-HSCT patient #37 was directed to an HLA-A2-restricted Taxll-19 epitope (Fig. 3). As for the HLA-A2 restricted Taxll-19 epitope, the present inventors have previously reported these as the dominant epitope of CTL restricted by human HLA-A2 (J. Virol. 66, 2928-2933, 1992).

### B-3 (Different HTLV-I-specific responses among pre-HSCT patient, post-HSCT patient, and donor)

It was next investigated whether there are any qualitative or quantitative differences in HTLV-I-specific CTL responses among pre-HSCT #37, post-HSCT #37, and donor #36. To examine HTLV-I-specific CTL precursor cell number without experimental biases by in vitro culture, frozen stored PBMC were immediately subjected to ELISPOT assay for IFN-γ production. The results of CTL precursor in the uncultured PBMC of recipient #37 and donor #36 responding to ILT-#37 or Tax epitope are shown in Table 1. The frozen stored uncultured PBMC of pre-HSCT and post-HSCT patient #37 and the uncultured PBMC of donor #36 were thawed directly, and after an overnight incubation with formalin-treated ILT-#37, synthetic oligopeptides Tax11-19 and Tax307-315, or control medium, as described in the above section A [Methods and Materials], ELISPOT assay of IFN-γ was performed, to calculate the number of IFN-γ producing cells per 10⁵. Values represent the mean ± SD of triplicate assays. Further, the results of ELISPOT assay of IFN-γ are shown by spot forming cells (SFC) per 10⁵ PBMC. As a result, the number of IFN-γ-producing cells resulting from overnight stimulation with ILT-#37 was similar in post-HSCT patient #37 and the donor #36. However, the number of IFN-γ producing cells responding to Taxll-19 peptides was higher in post-HSCT #37 than in donor #36. In the PBMC of pre-HSCT patient #37, numbers of IFN-γ-producing cells were generally higher even without stimulation and were further increased by stimulation with ILG-#37 cells or Taxll-19 peptides.

**(Table 1)**

| | IFN-γ-producing SFC/10⁵ PBMC⁺ | | |
|---|---|---|---|
| Stimulator | Pre-HSCT #37 | Post-HSCT #37 | Donor #36 |
| ILT-#37 | 58 ± 10 | 17 ± 2 | 16 ± 1 |
| Taxll-19 (LLFGYPVYV) | 42 ± 18 | 18 ± 2 | 7 ± 3 |
| Tax307-315 (LLFEEYTNI) | 18 ± 2 | 2 ± 1 | 1 ± 0.6 |
| Medium | 29 ± 12 | 2 ± 1 | 1 ± 1 |

The present inventors then cultured the PBMC of pre-HSCT patient #37 in the presence of IL-2 with periodic stimulation by ILT-#37 cells. Unlike the PBMC of post-HSCT patient #37, pre-HSCT PBMC failed to multiply, and could not be maintained for more than 7 weeks. The cytotoxic ability of this cell line at 40 days after initiation of culture is displayed (Fig. 4a). In contrast to similarly cultured PBMC from post-HSCT #37 and donor #36 at 46 days of culture, the culture from pre-HSCT patient #37 exhibited no significant levels of HTLV-I-specific cytotoxicity. The present inventors also stained these cultured PBMC with HLA-A*0201/Tax11-19 tetramer. The proportion of HLA-A*0201/Tax11-19 cells among CD8⁺ cells was markedly higher in post-HSCT patient #37 than in donor #36 (Fig. 4b). The culture of pre-HSCT patient #37 mostly consisted of CDB-negative cells. These observations indicated that HTLV-I-specific CTLs of post-HSCT #37 and donor #36 but not those of pre-HSCT #37 were capable of proliferating in vivo in response to stimulation with ILT-#37, and that Taxll-19-specific CTL were selectively activated in post-HSCT patient #37.

### B-4 (Induction of HTLV-I-specific CTL following HSCT from an HTLV-I negative donor)

In the second cases of HSCT, cellular immune response of post-HSCT patient R07 (+255 days of HSCT) against ILT-R07 originating from pre-HSCT patient R07 was similarly investigated. In three weeks of in vitro culture, CD8⁺ cells-enriched PBMC from post-HSCT R07 patient stimulated with formaldehyde-treated ILT-R07 exhibited significant levels of cytotoxicities and IFN-γ production against ILT-R07 and allogeneic HTLV-I-infected cells that shared HLA-A24. The presence of HLA-A24-restricted HTLV-I-specific CT1 in the PBMC culture of post-HSCT R07 patient was thus strongly suggested. Subsequently, epitope mapping of these CTL was performed. Of the panel of 15 to 24 mer oligopeptides of Tax and five 9-mer oligopeptides, the most probable HLA-A24-restricted epitopes as predicted by a computer program, Tax301-315 (SFHSLHLLFEEYTNI; SEQ ID NO: 3) and Tax301-309 (SFHSLHLLF; SEQ ID NO:4), were selectively reacted with the responder cells (Fig. 5). The present inventors stained these cultured PBMCs with the tetramer of HLA-A*2402/Tax301-309. The ratio of HLA-A*2402/Tax301-309+ cells in CD8⁺ cells were more than 30%. These observations indicated that HTLV-I-specific CTL response to selective Tax epitopes was induced in post-HSCT patient R07, as observed in the post-HSCT patient #37.

### C [Results]

In two ATL patients, HTLV-I-Tax-specific CTL response to a limited number of epitopes was observed following non-myeloablative HSCT from HLA-identical siblings. The PBMC culture from post-HSCT patient #37 contained a much larger number of CTL stained with HLA-A*0201/Tax11-19 tetramer than the CTL line from HTLV-I-carrying donor #36 (Fig. 4b). This implies that the HTLV-I-specific CTL response in post-HSCT patient differed from that in the donor not only in quantity but also in quality. In the donor, HTLV-I-specific T cell immune response must have been evoked as an acquired host defense response against primary HTLV-I infection. In contrast, in the recipient following HSCT, T cell immunity was introduced in the state of persistent HTLV-I-infection. It is intriguing that similar oligoclonal expansion of HTLV-I-specific CTL observed in recipient after transplantation is observed in HAM/TSP patients whose viral load is generally high (Virology 217, 139-146, 1996; J. din. Invest. 94, 1830-1839, 1994), suggesting that the particular pattern of HTLV-I-specific response observed in the post-HSCT ATL patients might be due to abundant antigen presentation in vivo. In the second case of HSCT from an uninfected donor, CTL induced from the post-HSCT patient R07 also recognized specifically a limited epitope, that is Tax301-309 epitope restricted by HLA-A24, indicating that the reason for the selective CTL response is in the recipient but not in the donor side.

Uncultured PBMC of post-HSCT patient #37 and donor #36 contained similar numbers of IGN-y-producing cells detected by ELISPOT assay after overnight stimulation with HTLV-I antigens (Table 1). Notably, the PBMC of patient #37 before HSCT included significant numbers of spontaneously IFN-γ-producing cells even without stimulation. However, following in vitro culture with stimulation with ILT-#37 cells, HTLV-I-specific CTL could be induced from post-HSCT patient #37 and donor #36, but not from pre-HSCT patient #37 (Figs. 4a, b), consistently with the previous observation that outgrowth of HTLV-I-specific CTL is rare in ATL patients (Leukemia 8 Suppl 1, S54-59, 1994; J. Immunol. 133, 1037-1041, 1984; J. Exp. Med. 177, 1567-1573, 1993; Proc. Natl. Acad. Sci. USA 95, 7568-7573, 1998; J. Immunol. 162, 1765-1771, 1991). Although the reasons for the failure of HTLV-I-specific CTL to proliferate in ATL patients is still unclear, the results obtained by the present inventors indicated that immune reconstitution following HSCT rendered HTLV-I-specific CTL in a memory state capable of proliferating upon antigen stimulation.

Several mHA that have been suggested to be involved in GVHD (N. Engl. J. Med. 334, 281-285, 1996; Science 279, 1054-1057, 1998; Br. J. Haematol 106, 723-729, 1999; Blood 92, 2169-2176, 1998) are candidates for GVL-targets. ILT-#37 and ILT-R07 cell lines originated from the ATL patients before HSCT possessed antigens of recipient origin as well as HTLV-I antigen. The cytotoxicity of CTL line established from post-HSCT patient #37 against ILT-#37 was not completely competed even by using unlabeled Tax-expressing cells (Fig. 2b), indicating the concurrent presence of CTL recognizing other antigens besides Tax-specific ones. The exact target antigens for GVL effects and contribution of HTLV-I Tax specific CTL to GVL effects remain to be clarified. However, the fact that strong and selective HTLV-I-specific CTL responses similarly observed in HAM/TSP patients were established in ATL patients following allogeneic HSCT from HLA-identical siblings shows that CTL epitope was strongly expressed in the patient body and suggests that CTL epitope described herein is useful as vaccine antigen. According to this vaccine antigen, it becomes possible to induce CTL suppressing HTLV-I infected cells proliferation in vivo.
(1) The dominant epitope of CTL restricted by HLA-A24 that 60% or more of the Japanese have, has been found. By using the peptide of the present epitope site for examination of immune response to HTLV-I, it is possible to cover most of the Japanese group.
(2) Presently, it is possible to estimate the epitope from amino acid anchor motif having affinity for each HLA. However, the host immune response to pathogens in the living body does not always correspond to this estimation. The epitopes identified herein are obtained from infected individuals, and are recognized with extremely strong selectivity compared to other epitopes.
(3) It is rare that HTLV-I-specific CTL is induced from ATL patients. From ATL cases completely remitted after stem cells transplantation, CTL to epitopes identified herein were selectively induced. This shows that the epitope was strongly expressed in the patient body and that the epitope is useful as vaccine antigen.

### SEQUENCE LISTING

<110> JAPAN SCIENSE AND TECHNOLOGY CORPORATION
<120> Peptide having activity deriving HTLV-I-specific CTL
<130> P018P01US(PCT)
<150> JP2003-112138
   <151> 2003-04-16
<160> 8
<170> PatentIn version 3.1
<210> 1
   <211> 9
   <212> PRT
   <213> Human T-cell lymphotropic virus type 1
<400> 1
   Leu Leu Phe Gly Tyr Pro Val Tyr Val
   1 5
<210> 2
   <211> 24
   <212> PRT
   <213> Human T-cell lymphotropic virus type 1
<400> 2
   Met Ala His Phe Pro Gly Phe Gly Gln Ser Leu Leu Phe Gly Tyr Pro
   1 5 10 15
   Val Tyr Val Phe Gly Asp Cys Val
   20
<210> 3
   <211> 15
   <212> PRT
   <213> Human T-cell lymphotropic virus type 1
<400> 3
   Ser Phe His Ser Leu His Leu Leu Phe Glu Glu Tyr Thr Asn Ile
   1 5 10 15
<210> 4
   <211> 9
   <212> PRT
   <213> Human T-cell lymphotropic virus type 1
<400> 4
   Ser Phe His Ser Leu His Leu Leu Phe
   1 5
<210> 5
   <211> 27
   <212> DNA
   <213> Human T-cell lymphotropic virus type 1
<400> 5
   cttcttttcg gatacccagt ctacgtg 27
<210> 6
   <211> 72
   <212> DNA
   <213> Human T-cell lymphotropic virus type 1
<400> 6
   atggcccact tcccagggtt tggacagagt cttcttttcg gatacccagt ctacgtgttt 60
   ggagactgtg ta 72
<210> 7
   <211> 45
   <212> DNA
   <213> Human T-cell lymphotropic virus type 1
<400> 7
   tcctttcata gtttacatct cctgtttgaa gaatacacca acatc 45
<210> 8
   <211> 27
   <212> DNA
   <213> Human T-cell lymphotropic virus type 1
<400> 8
   tcctttcata gtltacatct cctgttt 27

## Claims

1. A peptide having HTLV-I-specific CTL-inducing activity consisting of the amino acid sequence shown in SEQ ID NO: 4.

2. A peptide having HTLV-I-specific CTL-inducing activity consisting of an amino acid sequence wherein one amino acid is deleted, substituted or added in the amino acid sequence shown in SEQ ID NO: 4.

3. A fusion peptide wherein the peptide having HTLV-I-specific CTL-inducing activity according to claim 1 or 2 is bound to a marker protein and/or a peptide tag.

4. A peptide fusion HLA-A24 protein wherein the peptide having HTLV-I specific CTL-inducing activity according to Claim 1 or 2 is bound to HLA-A24 protein.

5. A tetramer of the peptide fusion HLA-A24 protein according to claim 4.

6. A fusion protein wherein the peptide HLA-A24 fusion protein according to claim 4 or the tetramer according to claim 5 is bound to a marker protein and/or a peptide tag.

7. A DNA encoding the following peptide (a) or (b); (a) a peptide having HTLV-I-specific CTL-inducing activity consisting of the amino acid sequence shown in SEQ ID NO: 4, (b) a peptide having HTLV-I-specific CTL-inducing activity consisting of an amino acid sequence wherein one amino acid is deleted, substituted or added in the amino acid sequence shown in SEQ ID NO: 4.

8. A DNA consisting of the base sequence shown in SEQ ID NO: 8, or a complementary sequence thereof.

9. A HLA-A24-restricted Tax-epitope consisting of a peptide having HTLV-I-specific CTL-inducing activity consisting of the amino acid sequence shown in SEQ ID NO: 4.

10. A vaccine to induce immune response containing a peptide having HTLV-I-specific CTL-inducing activity consisting of the amino acid sequence shown in SEQ ID NO: 4, as active ingredient.

11. A vaccine to induce immune response containing a peptide having HTLV-I-specific CTL-inducing activity consisting of an amino acid sequence wherein one amino acid is deleted, substituted or added in the amino acid sequence shown in SEQ ID NO: 4, as active ingredient.

12. A vaccine to induce immune response containing a vector capable of expressing the DNA according to claim 7 or 8, as active ingredient.

13. A vaccine to induce immune response according to any one of claims 10 to 12, comprising an adjuvant which enhances HTLV-I-specific CTL-inducing activity.

14. A medical composition containing the vaccine to induce immune response according to any one of claims 10 to 13, as active ingredient.

15. A diagnostic agent to examine immune function containing a peptide having HTLV-I-specific CTL-inducing activity consisting of the amino acid sequence shown in SEQ ID NO: 4, as active ingredient.

16. A diagnostic agent to examine immune function containing a peptide having HTLV-I-specific CTL-inducing activity consisting of an amino acid sequence, wherein one amino acid is deleted, substituted or added in the amino acid sequence shown in SEQ ID NO: 4, as active ingredient.

17. A diagnostic agent to examine immune function containing a vector capable of expressing the DNA according to claim 7 or 8, as active ingredient.

18. A diagnostic agent to examine immune function containing the peptide HLA-A24 fusion protein according to claim 4, as active ingredient.

19. A diagnostic agent to examine immune function containing a tetramer of the peptide HLA-A24 fusion protein according to claim 4, as active ingredient.

20. A diagnostic agent of HTLV-I tumor containing the DNA according to claim 7 or 8, as active ingredient.

21. An antibody binding specifically to the peptide having HTLV-I-specific CTL-inducing activity according to claim 1 or 2.

22. The antibody according to claim 21, wherein the antibody is a monoclonal antibody.

23. A diagnostic agent of HTLV-I tumor containing the antibody binding specifically to the peptide having HTLV-I-specific CTL-inducing activity according to claim 21 or 22, as active ingredient.

24. An expression vector capable of expressing the peptide having HTLV-I-specific CTL-inducing activity according to claim 1 or 2.

25. A host cell comprising an expression system capable of expressing the peptide having HTLV-I-specific CTL-inducing activity according to claim 1 or 2.

26. An expression vector capable of expressing the peptide HLA-A24 fusion protein according to claim 4.

27. A host cell comprising an expression system capable of expressing the peptide HLA-A24 fusion protein according to claim 4.

28. A peptide having HTLV-I-specific CTL-inducing activity according to claim 1 or 2 for use in a method for inducing HTLV-I recognizing CTL, the method comprising using the peptide to stimulate PBMC of an HLA-A24 positive ATL patient.

29. A vector capable of expressing the DNA according to claim 7 or 8 for use in a method for inducing HTLV-I recognizing CTL, the method comprising using the vector to stimulate PBMC of an HLA-A24 positive ATL patient.

## Patentansprüche

1. Peptid mit HTLV-I-spezifischer CTL-induzierender Aktivität, bestehend aus der in SEQ ID NO: 4 gezeigten Aminosäuresequenz.

2. Peptid mit HTLV-I-spezifischer CTL-induzierender Aktivität, bestehend aus einer Aminosäuresequenz, wobei in der in SEQ ID NO: 4 gezeigten Aminosäuresequenz eine Aminosäure deletiert, substituiert oder hinzugefügt ist.

3. Fusionspeptid, wobei das Peptid mit HTLV-1-spezifischer CTL-induzierender Aktivität nach Anspruch 1 oder 2 an ein Markerprotein und/oder eine Peptidmarkierung gebunden ist.

4. Peptid-HLA-A24-Fusionsprotein, wobei das Peptid mit HTLV-I-spezifischer CTL-induzierender Aktivität nach Anspruch 1 oder 2 an das HLA-A24-Protein gebunden ist.

5. Tetramer des Peptid-HLA-A24-Fusionsproteins nach Anspruch 4.

6. Fusionsprotein, wobei das Peptid-HLA-A24-Fusionsprotein nach Anspruch 4 oder das Tetramer nach Anspruch 5 an ein Markerprotein und/oder eine Peptidmarkierung gebunden ist.

7. DNA, welche die nachfolgenden Peptide (a) oder (b) codiert: (a) ein Peptid mit HTLV-1-spezifischer CTL-induzierender Aktivität, bestehend aus der in SEQ ID NO: 4 gezeigten Aminosäuresequenz, (b) ein Peptid mit HTLV-I-spezifischer CTL-induzierender Aktivität, bestehend aus einer Aminosäuresequenz, wobei in der in SEQ ID NO: 4 gezeigten Aminosäuresequenz eine Aminosäure deletiert, substituiert oder hinzugefügt ist.

8. DNA, bestehend aus der in SEQ ID NO: 8 gezeigten Basensequenz oder einer dazu komplementären Sequenz.

9. HLA-A24-restringiertes Tax-Epitop, bestehend aus einem Peptid mit HTLV-I-spezifischer CTL-induzierender Aktivität, welches aus der in SEQ ID NO: 4 gezeigten Aminosäuresequenz besteht.

10. Impfstoff zum Induzieren einer Immunantwort, welcher ein Peptid mit HTLV-I-spezifischer CTL-induzierender Aktivität, das aus der in SEQ ID NO: 4 gezeigten Aminosäuresequenz besteht, als aktiven Inhaltsstoff enthält.

11. Impfstoff zum Induzieren einer Immunantwort, welcher ein Peptid mit HTLV-1-spezifischer CTL-induzierender Aktivität, das aus einer Aminosäuresequenz besteht, wobei in der in SEQ ID NO: 4 gezeigten Aminosäuresequenz eine Aminosäure deletiert, substituiert oder hinzugefügt ist, als aktiven Inhaltsstoff enthält.

12. Impfstoff zum Induzieren einer Immunantwort, welcher einen Vektor, der die DNA nach Anspruch 7 oder 8 exprimieren kann, als aktiven Inhaltsstoff enthält.

13. Impfstoff zum Induzieren einer Immunantwort nach einem der Ansprüche 10 bis 12, welcher ein Adjuvans umfaßt, das die HTLV-I-spezifische CTL-induzierende Aktivität verstärkt.

14. Medizinische Zusammensetzung, welche den Impfstoff zum Induzieren einer Immunantwort nach einem der Ansprüche 10 bis 13 als aktiven Inhaltsstoff enthält.

15. Diagnostisches Mittel zum Untersuchen der Immunfunktion, welches ein Peptid mit HTLV-I-spezifischer CTL-induzierender Aktivität, bestehend aus der in SEQ ID NO: 4 gezeigten Aminosäuresequenz, als aktiven Inhaltsstoff enthält.

16. Diagnostisches Mittel zum Untersuchen der Immunfunktion, welches ein Peptid mit HTLV-I-spezifischer CTL-induzierender Aktivität, bestehend aus einer Aminosäuresequenz, wobei in der in SEQ ID NO: 4 gezeigten Aminosäuresequenz eine Aminosäure deletiert, substituiert oder hinzugefügt ist, als aktiven Inhaltsstoff enthält.

17. Diagnostisches Mittel zum Untersuchen der Immunfunktion, welches einen Vektor, der die DNA nach Anspruch 7 oder 8 exprimieren kann, als aktiven Inhaltsstoff enthält.

18. Diagnostisches Mittel zum Untersuchen der Immunfunktion, welches das Peptid-HLA-A24-Fusionsprotein nach Anspruch 4 als aktiven Inhaltsstoff enthält.

19. Diagnostisches Mittel zum Untersuchen der Immunfunktion, welches ein Tetramer des Peptid-HLA-A24-Fusionsproteins nach Anspruch 4 als aktiven Inhaltsstoff enthält.

20. Diagnostisches Mittel für einen HTLV-I-Tumor, welches die DNA nach Anspruch 7 oder 8 als aktiven Inhaltsstoff enthält.

21. Antikörper, welcher spezifisch an das Peptid mit HTLV-1-spezifischer CTL-induzierender Aktivität nach Anspruch 1 oder 2 bindet.

22. Antikörper nach Anspruch 21, wobei der Antikörper ein monoklonaler Antikörper ist.

23. Diagnostisches Mittel für einen HTLV-I-Tumor, welches den Antikörper, der spezifisch an das Peptid mit HTLV-I-spezifischer CTL-induzierender Aktivität bindet, nach Anspruch 21 oder 22 als aktiven Inhaltsstoff enthält.

24. Expressionsvektor, welcher das Peptid mit HTLV-I-spezifischer CTL-induzierender Aktivität nach Anspruch 1 oder 2 exprimieren kann.

25. Wirtszelle, die ein Expressionssystem, welches das Peptid mit HTLV-I-spezifischer CTL-induzierender Aktivität nach Anspruch 1 oder 2 exprimieren kann, umfaßt.

26. Expressionsvektor, welcher das Peptid-HLA-A24-Fusionsprotein nach Anspruch 4 exprimieren kann.

27. Wirtszelle, die ein Expressionssystem, welches das Peptid-HLA-A24-Fusionsprotein nach Anspruch 4 exprimieren kann, umfaßt.

28. Peptid mit HTLV-I-spezifischer CTL-induzierender Aktivität nach Anspruch 1 oder 2 zur Verwendung in einem Verfahren zum Induzieren von HTLV-I-erkennenden CTL, wobei das Verfahren die Verwendung des Peptids zum Stimulieren von PBMC eines HLA-A24-positiven ATL-Patienten umfaßt.

29. Vektor, welcher die DNA nach Anspruch 7 oder 8 exprimieren kann, zur Verwendung in einem Verfahren zum Induzieren von HTLV-I-erkennenden CTL, wobei das Verfahren die Verwendung des Vektors zum Stimulieren von PBMC eines HLA-A24-positiven ATL-Patienten umfaßt.

## Revendications

1. Peptide ayant une activité d'induction de CTL spécifique du HTLV-I, consistant en la séquence d'aminoacides représentée dans la SEQ ID N° 4.

2. Peptide ayant une activité d'induction de CTL spécifique du HTLV-I, consistant en une séquence d'aminoacides dans laquelle un aminoacide est éliminé par délétion de, substitué dans, ou ajouté à la séquence d'aminoacides représentée dans la SEQ ID N° 4.

3. Peptide de fusion, dans lequel le peptide ayant une activité d'induction de CTL spécifique du HTLV-I suivant la revendication 1 ou 2 est lié à une protéine servant de marqueur et/ou à un marqueur peptidique.

4. Peptide de fusion de protéine HLA-A24, lequel peptide ayant une activité d'induction de CTL spécifique du HTLV-I suivant la revendication 1 ou 2 est lié à la protéine HLA-A24.

5. Tétramère du peptide de fusion de protéine HLA-A24 suivant la revendication 4.

6. Protéine de fusion, dans laquelle le peptide de fusion de protéine HLA-A24 suivant la revendication 4 ou le tétramère suivant la revendication 5 est lié à une protéine servant de marqueur et/ou un marqueur peptidique.

7. ADN codant pour le peptide (a) ou (b) suivant : (a) un peptide ayant une activité d'induction de CTL spécifique du HTLV-I, consistant en la séquence d'aminoacides représentée dans la SEQ ID N° 4, (b) un peptide ayant une activité d'induction de CTL spécifique du HTLV-I, consistant en une séquence d'aminoacides dans laquelle un aminoacide est éliminé par délétion de, substitué dans, ou ajouté à la séquence d'aminoacides représentée dans la SEQ ID N° 4.

8. ADN consistant en la séquence de base représentée dans la SEQ ID N° 8, ou une séquence complémentaire de celle-ci.

9. Epitope Tax à restriction HLA-A24, consistant en un peptide ayant une activité d'induction de CTL spécifique du HTLV-I, consistant en la séquence d'aminoacides représentée dans la SEQ ID N° 4.

10. Vaccin pour induire une réponse immunitaire, contenant un peptide ayant une activité d'induction de CTL spécifique du HTLV-I, consistant en la séquence d'aminoacides représentée dans la SEQ ID N° 4, comme ingrédient actif.

11. Vaccin pour induire une réponse immunitaire, contenant un peptide ayant une activité d'induction de CTL spécifique du HTLV-I, consistant en une séquence d'aminoacides dans laquelle un aminoacide est éliminé par délétion de, substitué dans, ou ajouté à la séquence d'aminoacides représentée dans la SEQ ID N° 4, comme ingrédient actif.

12. Vaccin pour induire une réponse immunitaire, contenant un vecteur capable d'exprimer l'ADN suivant la revendication 7 ou 8, comme ingrédient actif.

13. Vaccin pour induire une réponse immunitaire suivant l'une quelconque des revendications 10 à 12, comprenant un adjuvant qui augmente l'activité d'induction de CTL spécifique du HTLV-I.

14. Composition médicale contenant le vaccin pour induire une réponse immunitaire suivant l'une quelconque des revendications 10 à 13, comme ingrédient actif.

15. Agent de diagnostic pour étudier la fonction immunitaire, contenant un peptide ayant une activité d'induction de CTL spécifique du HTLV-I, consistant en la séquence d'aminoacides représentée dans la SEQ ID N° 4, comme ingrédient actif.

16. Agent de diagnostic pour étudier la fonction immunitaire, contenant un peptide ayant une activité d'induction de CTL spécifique du HTLV-I, consistant en une séquence d'aminoacides, dans laquelle un aminoacide est éliminé par délétion de, substitué dans, ou ajouté à la séquence d'aminoacides représentée dans la SEQ ID N° 4, comme ingrédient actif.

17. Agent de diagnostic pour étudier la fonction immunitaire, contenant un vecteur capable d'exprimer l'ADN suivant la revendication 7 ou 8, comme ingrédient actif.

18. Agent de diagnostic pour étudier la fonction immunitaire, contenant le peptide de fusion de protéine HLA-A24 suivant la revendication 4, comme ingrédient actif.

19. Agent de diagnostic pour étudier la fonction immunitaire, contenant un tétramère du peptide de fusion de protéine HLA-A24 suivant la revendication 4, comme ingrédient actif.

20. Agent de diagnostic d'une tumeur provoquée par le HTLV-I, contenant l'ADN suivant la revendication 7 ou 8, comme ingrédient actif.

21. Anticorps se liant spécifiquement au peptide ayant une activité d'induction de CTL spécifique du HTLV-I suivant la revendication 1 ou 2.

22. Anticorps suivant la revendication 21, l'anticorps étant un anticorps monoclonal.

23. Agent de diagnostic d'une tumeur provoquée par le HTLV-I, contenant l'anticorps se liant spécifiquement au peptide ayant une activité d'induction de CTL spécifique du HTLV-I suivant la revendication 21 ou 22, comme ingrédient actif.

24. Vecteur d'expression apte à l'expression du peptide ayant une activité d'induction de CTL spécifique du HTLV-I suivant la revendication 1 ou 2.

25. Cellule hôte comprenant un système d'expression apte à l'expression du peptide ayant une activité d'induction de CTL spécifique du HTLV-I suivant la revendication 1 ou 2.

26. Vecteur d'expression apte à l'expression du peptide de fusion de protéine HLA-A24 suivant la revendication 4.

27. Cellule hôte comprenant un système d'expression apte à l'expression du peptide de fusion de protéine HLA-A24 suivant la revendication 4.

28. Peptide ayant une activité d'induction de CTL spécifique du HTLV-I suivant la revendication 1 ou 2, destiné à être utilisé dans une méthode pour induire des CTL reconnaissant le HTLV-I, méthode comprenant l'utilisation du peptide pour stimuler les PBMC d'un patient ATL positif pour HLA-A24.

29. Vecteur apte à l'expression de l'ADN suivant la revendication 7 ou 8, destiné à être utilisé dans une méthode pour induire des CTL reconnaissant le HTLV-I, méthode comprenant l'utilisation du vecteur pour stimuler les PBMC d'un patient ATL positif pour HLA-A24.
